# EUROPEAN PATENT APPLICATION

(11) **EP 1 816 208 A1**
(43) Date of publication of application: **08.08.2007**
(21) Application number: 05809323.8
(22) Date of filing: 16.11.2005
(51) Int. Cl.: C12Q 1/00, G01N 33/50, G01N 33/15

(54) **METHOD OF SCREENING CANDIDATE FOR DRUG**

(30) Priority: 16.11.2004 JP 2004332376
(71) Applicant: Hiroshima Industrial Promotion Organization, Hiroshima-shi, Hiroshima 730-0052 (JP); BIOINTEGRENCE INC., Hiroshima-shi, Hiroshima 733-0013 (JP); Phoenixbio Co., Ltd., Higashihiroshima-shi, Hiroshima 739-0046 (JP); SUMIKA CHEMICAL ANALYSIS SERVICE, LIMITED, Osaka-shi Osaka 554-0022 (JP)
(72) Inventor: MUKAIDANI, Chise, Higashihiroshima-shi, Hiroshima 739-0024 (JP); YOSHIZATO, Katsutoshi, Higashihiroshima-shi, Hiroshima 739-0144 (JP); NISHIKURA, Yasufumi c/o Phoenixbio Co., Ltd., Higashihiroshima-shi, Hiroshima 739-0046 (JP); HORIE, Toru c/o Phoenixbio Co., Ltd., Higashihiroshima-shi, Hiroshima 739-0046 (JP); NAKAZAWA, Hiroshi Sumika Chem. Analysis,Serv. Ltd, Osaka-shi, 554-0022 (JP); KANAMARU, Hiroshi Sumika Chem. Analysis,Serv. Ltd, Osaka-shi, Osaka 554-0022 (JP); UEDA, Chiaki Sumika Chem. Analysis,Serv. Ltd., Osaka-shi, Osaka 554-0022 (JP); HINE, Chiemi Sumika Chemical Analysis,Serv. Ltd., Osaka-shi, Osaka 554-0022 (JP)
(74) Representative: Dzieglewska, Hanna Eva
(86) International application number: PCT/JP2005/021393
(87) International publication number: WO 2006/054755

(57) **Abstract**

It is intended to provide a method of screening a candidate drug mainly metabolized by a drug metabolizing enzyme, CYP2D6, CYP2C9 or CYP2C19 with the use of a chimeric mouse carrying human hepatocytes transplanted thereinto, whereby it is determined whether or not the candidate drug is metabolized in a subject deficient in CYP2D6, CYP2C9 or CYP2C19. It is also intended to provide a method of determining whether or not a candidate drug induces or inhibits the activity of any of drug metabolizing enzymes, CYP1A2, CYP2C9, CYP2C19, CYP2D6 and CYP3A4 with the use of a chimeric mouse carrying human hepatocytes transplanted thereinto.

## Description

### Technical Field

The invention of this application relates to a method of screening a candidate drug which is metabolized mainly by a drug-metabolizing enzyme, CYP2D6, CYP2C9 or CYP2C19. It also relates to a method of determining whether or not a candidate drug induces or inhibits the activity of any of drug-metabolizing enzymes of the CYP family.

### Background Art

In the development of a drug, a stage of selecting a candidate drug is of the highest interest to pharmaceutical companies. It is a well-known fact that the development of a drug requires enormous research and development costs of 30 billions or more and enormous periods of time of 10 years or more. Therefore, it must be avoided that the development of a candidate drug is ceased in the course of research and development. Further, Japanese domestic pharmaceutical companies have advanced global clinical development in Japan, US and Europe recently, and racial differences or ethnic differences in the effectiveness become obstacles in the global clinical development, therefore, the current situation is that companies avoid the development of a candidate drug showing racial differences. In general, as a factor to cause racial differences in pharmacokinetics, racial differences in a drug-metabolizing enzyme are conceived, and in particular, genetic polymorphisms of a drug-metabolizing enzyme, Cytochrome P450 (CYP) 2D6, CYP2C9 or CYP2C19 are considered to cause racial differences in pharmacokinetics of a candidate drug which is metabolized mainly by such an enzyme. For example, with regard to CYP2D6, there are about 10% of patients deficient in CYP2D6 (PM: poor metabolizer) in Westerners, therefore, the current situation is that compounds which are metabolized mainly by CYP2D6 are not selected as a candidate drug.

Further, drug interaction of medications becomes a critical issue in the medical field because serious symptoms are caused in some combination of drugs. In the case where a problem involved in drug interaction takes place, it sometimes results in the event that the pharmaceutical company has to recall the drug from the market. As in the above, because the drug interaction based on the induction or inhibition of a drug-metabolizing enzyme becomes a key factor in decision-making in research and development of a drug, the prediction of drug interaction in human is of an important interest to the research and development.

Incidentally, the inventors of this application succeeded in producing a chimeric mouse in which human hepatocytes associated with drug metabolism in human have been replaced with human hepatocytes at a high ratio in the mouse liver, and applied for a patent (Patent documents 1 and 2).
Patent document 1: Japanese Patent Publication No. 2002-45087
Patent document 2: International Patent Publication No. WO 2003/080821

### Disclosure of the invention

However, it is difficult to determine whether or not it is a candidate drug which is metabolized mainly by CYP2D6, CYP2C9 or CYP2C19 at a stage in which a labeled compound with a radioisotope is not available. When an ADME test is carried out using a labeled compound for a candidate drug, the entire metabolic pathway will be elucidated, and what percentage the metabolic process associated with CYP2D6, CYP2C9 or CYP2C19 account for in the metabolism of the candidate drug will be found. Thus, in a situation in which a labeled compound is not available in a search stage; the involvement of CYP2D6, CYP2C9 or CYP2C19 metabolism in the candidate drug cannot be evaluated accurately.

The invention of this application has been made in view of the above circumstances, and has it object to provide a screening method in which when a candidate drug is a substance metabolized mainly by CYP2D6, CYP2C9 or CYP2C19, whether or not genetic polymorphisms affect the pharmacokinetics is determined.

Further, there has been no. in vivo system for detecting drug interaction in human so far. In an in vitro system, it was difficult to predict drug interaction in the human body because the drug exposure level to cells was always high. Thus, an object of the invention of this application is to provide a screening method for detecting drug interaction in human in vivo.

This application provides as a first invention for achieving the above object, a method of determining whether or not a candidate drug metabolized mainly by a drug-metabolizing enzyme, CYP2D6, CYP2C9 or CYP2C19 is metabolized in a subject deficient in CYP2D6, CYP2C9 or CYP2C19, which comprises the steps of:
(1) administering the candidate drug to a high-replacement chimeric mouse in which transplanted human hepatocytes account for 70% or more of the mouse liver, and a low-replacement chimeric mouse in which few human hepatocytes are engrafted, respectively; and
(2) measuring the time-course concentration of the candidate drug in serum collected from the high-replacement chimeric mouse and the low-replacement chimeric mouse,
   and determining that the candidate drug which is metabolized significantly more quickly in the high-replacement chimeric mouse than in the low-replacement chimeric mouse is a substance which is not metabolized in the subject deficient in CYP2D6, CYP2C9 or CYP2C19.

This application also provides as a second invention, a method of determining whether or not a candidate drug inducesor inhibits the activity of a drug-metabolizing enzyme, which comprises the steps of:
(1) administering the candidate drug to a high-replacement chimeric mouse;
(2) administering a plurality of marker compounds which are metabolized by a respective plurality of drug-metabolizing enzymes to the high-replacement chimeric mouse; and
(3) measuring the time-course concentrations of the marker compounds in serum collected from the high-replacement chimeric mouse,
   and determining whether or not the candidate drug induces or inhibits the activity of any of the drug-metabolizing enzymes by the comparison with the concentrations of marker compounds in serum when the candidate drug is not administered.

### Brief Description of the Drawings

Fig. 1 is a view showing examples of peaks detected in an analysis by LC-MS/MS of serum samples from mice to which 5 compounds were simultaneously administered.
Fig. 2 shows the time course changes in the concentration of caffeine detected in mouse serum when caffeine was administered to mice. Chimeric mice transplanted with donor cells (IVT079) were used.
Fig. 3 shows the time course changes in the concentration of tolbutamide detected in mouse serum when tolbutamide was administered to mice. Chimeric mice transplanted with donor cells (IVT079) were used.
Fig. 4 shows the time course changes in the concentration of omeprazole detected in mouse serum when omeprazole was administered to mice. Chimeric mice transplanted with donor cells (IVT079) were used.
Fig. 5 shows the time course changes in the concentration of dextromethorphan detected in mouse serum when dextromethorphan was administered to mice. Chimeric mice transplanted with donor cells (IVT079) were used.
Fig. 6 shows the time course changes in the concentration of erythromycin detected in mouse serum when erythromycin was administered to mice. Chimeric mice transplanted with donor cells (IVT079) were used.
Fig. 7 shows the time course changes in the concentration of caffeine or tolbutamide detected in mouse serum when caffeine or tolbutamide was administered to mice. Chimeric mice transplanted with donor cells (BD51) were used.
Fig. 8 shows the time course changes in the concentration of omeprazole or dextromethorphan detected in mouse serum when omeprazole or dextromethorphan was administered to mice. Chimeric mice transplanted with donor cells (BD51) were used.
Fig. 9 shows the time course changes in the concentration of erythromycin detected in mouse serum when erythromycin was administered to mice. Chimeric mice transplanted with donor cells (BD51) were used.
Fig. 10 shows the time course changes in the concentration of caffeine, tolbutamide, omeprazole, dextromethorphan or erythromycin detected in serum from chimeric mice with high replacement when each of the substances was administered to the mice after administration of paroxetine or without administration of paroxetine. Chimeric mice transplanted with donor cells (BD51) were used.

### Best Mode for Carrying Out the Invention

A method of the first invention comprises the steps of:
(1) administering a candidate drug to a high-replacement chimeric mouse and a low-replacement chimeric mouse, respectively; and
(2) measuring the time-course concentration of the candidate drug in serum collected from the high-replacement chimeric mouse and the low-replacement chimeric mouse. And the method is characterized by determining that the candidate drug which is metabolized significantly more quickly in the high-replacement chimeric mouse than in the low-replacement chimeric mouse is a substance which is not metabolized in the subject deficient in CYP2D6, CYP2C9 or CYP2C19.

In the high-replacement chimeric mouse, transplanted human hepatocytes account for 70% or more of the mouse liver. The mouse can be obtained by the method described in the Patent document 1 or 2. In particular, the method described in the Patent document 2 (a method of raising a mouse in a condition in which the cells are protected from the attack of human complement produced by the transplanted human hepatocytes) is preferred as a method of achieving the replacement ratio of human hepatocytes of 70% or more. Alternatively, in the low-replacement chimeric mouse, few human hepatocytes are engrafted (more specifically, the replacement ratio of human hepatocytes is less than 1%).

These chimeric mice are used, for example, for administration of a candidate drug after around 60 days from the transplantation of human hepatocytes. Incidentally, the replacement ratio of human hepatocytes in the chimeric mouse can be confirmed in advance, by a method such as measurement of the concentration of human albumin in the mouse blood. Further, a more accurate replacement ratio can be confirmed by an anatomical examination of the liver of the chimeric mouse after the test using the method described in the Patent document 2 or the like.

The candidate drug is a substance in the course of development of a drug and requires prediction of drug interaction in human. Such a substance may be a substance of major ingredient for a drug efficacy, or may be a composition containing a substance of major ingredient. The administration amount of the candidate drug varies depending on the type of disease for which the substance is intended, the type of the substance composition, administration route or the like, however, it can be set to about 0.1 mg/kg of body weight to 2000 mg/kg of body weight. Further, as the administration route, oral, subcutaneous, intravenous or intraperitoneal administration or the like can be employed according to the type of the candidate drug, a dosage form suitable for the substance.

The degree of metabolism of a candidate drug in the liver of a chimeric mouse can be determined by measuring the concentration of the candidate drug in the mouse serum by a standard method in this technical field (for example, chromatography described in Examples or the like). Further, the measurement can be carried out over time (at about 15 minutes to 24 hours intervals) from about 30 minutes to 24 hours after the candidate of a drug is administered.

Then, it is determined whether the candidate drug is a substance which is easily metabolized in a subject deficient in CYP2D6, CYP2C9 or CYP2C19 or a substance which is not easily metabolized in the subject based on the concentration (the concentration of the substance metabolized by the drug metabolizing enzyme in the liver) of the candidate drug in this mouse serum. More specifically, in the case where a candidate drug is metabolized significantly more quickly in the high-replacement chimeric mouse than in the low-replacement chimeric mouse, it is considered that the candidate drug is metabolized by CYP2D6, CYP2C9 or CYP2C19. Therefore, it is determined that this candidate drug is a substance which is not metabolized in a subject deficient in CYP2D6, CYP2C9 or CYP2C19.

Incidentally, the description of "being metabolized significantly more quickly" means a case in which an AUC (area under the curve) calculated based on the concentration of the candidate drug in the mouse serum is about 2/3 as large as that of the low-replacement chimeric mouse, and preferably an average AUC is about 1/2 to 1 / 3 as large as that of the low-replacement chimeric mouse.

The method of the second invention comprises the steps of:
(1) administering the candidate drug to a high-replacement chimeric mouse;
(2) administering a plurality of marker compounds which are metabolized by a respective plurality of drug-metabolizing enzymes to the high-replacement chimeric mouse; and
(3) measuring the time-course concentrations of the marker compounds in serum collected from the high-replacement chimeric mouse. And the method determines whether or not the candidate drug induces or inhibits the activity of any of the drug-metabolizing enzymes by the comparison with the concentrations of marker compounds in serum when the candidate drug is not administered.

Examples of the human drug metabolizing enzymes include CYP1A1, CYP1A2, CYP1B1, CYP2A6, CYP2B6, CYP2C8, CYP2C9, CYP2C10, CYP2C18, CYP2C19, CYP2D6, CYP2E1, CYP3A3, CYP3A4, CYP3A5, CYP3A7, CYP4F1, CYP4A2, CYP4A3, which are in the CYP family, and the like. Examples of the marker compounds for the respective enzymes include caffeine for CYP1A2, tolbutamide for CYP2C9, dextromethorphan for CYP2D6, omeprazole for CYP2C19, erythromycin for CYP3A4 and the like. After a candidate drug is administered to a high-replacement chimeric mouse, a mixture of these marker compounds are administered to the mouse, and the time-course concentrations of the respective compounds in the serum are measured, whereby it can be determined whether the candidate drug is a substance which induces the activity of any of the enzymes or a substance which inhibits the activity of any of the enzymes. For example, in the mixture of the marker compounds, when the metabolism of caffeine is promoted and the concentration thereof in the serum has decreased, it is determined that the candidate drug is a substance which inducesspecifically the activity of CYP1A2.

### Examples

Hereinafter, this invention will be described in more detail and specifically with reference to Examples, however, this invention is not limited to the following examples.

### Example 1

### Pharmacokinetic test using chimeric mouse

### 1. Materials and Methods

### 1-1. Test substance

In order to evaluate pharmacokinetics in human hepatocyte chimeric mice using marker drugs, 5 compounds, i.e., caffeine (CYP1A2), tolbutamide (CYP2C9), omeprazole (CYP2C19), dextromethorphan (CYP2D6) and erythromycin (CYP3A4) which are standard drugs for cytochrome P450 (CYP) were used.

### 1-2. Animals

As for the chimeric mice, chimeric mice produced by Hiroshima Prefectural Institute of Industrial Science and Technology or PhoenixBio Co., Ltd. were used. To be more specific, mice in which human hepatocytes were engrafted and proliferated in the mouse liver were produced by transplanting human hepatocytes (IVT079, purchased from In Vitro Technology Inc., or BD51, purchased from BD Gentest Inc.) into the spleen of uPA(+/+) / SCID mice, which are mice having hepatic impairment and immunodeficiency. Mice in which 70% or more of the mouse liver had been replaced with human hepatocytes (high-replacement chimeric mice) and low-replacement chimeric mice in which although human hepatocytes had been transplanted, the replacement ratio was almost 0%, or untransplanted mice (mice with no replacement) were used (3 mice in each group). Further, as a control, 3 uPA(-/-)/SCID mice were used.
Each of the mice was fed with water (containing 0.012% hypochlorous acid) and vitamin C-containing sterile solid feed (CRF-1, Oriental Yeast Co., Ltd.) ad libitum and raised under the condition of room temperature of 24 ± 2°C. Incidentally, to the chimeric mice with high replacement, an injection solution of nafamostat mesilate (0.3 mg/0.2 ml/body) was intraperitoneally administered twice daily.

### 1-3. Preparation of drug to be administered

Each of the drugs was prepared to have a concentration 5 times as high as the concentration for administration such that the final concentrations of the respective 5 compounds after mixing the compounds immediately before administration become the respective concentrations for administration. The concentration for administration of caffeine which is a standard drug for CYP1A2 was 25 mg/kg b.w., and 12.5 mg of caffeine was weighed out and suspended in 1 ml of a CMC liquid in an agate mortar, and a 12.5 mg/ml suspension which is 5 times as high as the concentration for administration was prepared. The concentration for administration of tolbutamide which is a standard drug for CYP2C9 was 8.35 mg/kg b.w., and 4.175 mg of tolbutamide was weighed out and suspended in 1 ml of a CMC liquid in an agate mortar, and a 4.175 mg/ml suspension which is 5 times as high as the concentration for administration was prepared. The concentration for administration of dextromethorphan which is a standard drug for CYP2D6 was 2.5 mg/kg b.w., and 20 mg of dextromethorphan hydrobromide monohydrate was weighed out and suspended in 16 ml of a CMC liquid in an agate mortar, and a 1.25 mg/ml suspension which is 5 times as high as the concentration for administration was prepared. The concentration for administration of omeprazole which is a standard drug for CYP2C19 was 8.35 mg/kg b.w., and one vial of omeprazole (20 mg) was dissolved in 4.76 ml of Milli-Q water, and a 4.2 mg/ml aqueous solution which is 5 times as high as the concentration for administration was prepared. The concentration for administration of erythromycin which is a standard drug for CYP3A4 was 125 mg/kg b.w., and one vial of erythromycin lactobionate (500 mg) was dissolved in 8 ml of Milli-Q water, and a 62.5 mg/ml aqueous solution which is 5 times as high as the concentration for administration was prepared. The prepared respective drugs were mixed in an amount of 1 ml each to give a final volume of 5 ml, and the resulting suspension was administered to the mice at a dose of 10 µl/g b.w.

### 1-4. Administration method and serum collection method

To the high-replacement chimeric mice, low-replacement chimeric mice (or mice with no replacement) and uPA(-/-)/SCID mice (3 mice in each group), 5 compounds were simultaneously administered orally by gavage or intravenously. The blood was collected from the tail vein of each mouse in an amount of 15 µl each at 0.25 (only intravenous administration), 0:5, 1, 2, 4 (only oral administration) and 8 hours after administration. Then, the blood was centrifuged at 3,500 rpm for 5 minutes, and the serum (supernatant) was collected and stored in a freezer at -30°C.

### 1-5. Determination and analysis of drugs in serum

The serum samples were transported to Sumika Chemical Analysis Service, Ltd. while keeping it in dry ice, and subjected to measurement by LC-MS/MS according to the following procedure.

### 1-5-1. Preparation of standard solutions of substances to be measured and I.S. standard solutions

Caffeine, tolbutamide, omeprazole, dextromethorphan hydrobromide monohydrate and erythromycin lactobionate were diluted with methanol and standard solutions at 2000, 1000, 500, 100, 50, 10, 5, 1, 0.5 and 0.1 ng/ml were prepared, respectively. Phenytoin was dissolved in methanol, and standard solutions at 10 and 1 mg/ml were prepared and used as I. S. standard solutions.

### 1-5-2. Measurement device and measurement conditions (LC-MS/MS)

With regard to a liquid chromatograph, SIL-HTC and LC-10A series (Shimadzu Corporation) were used, and as a column, Synergi 4µ Polar-RP 80A, 150 mm (length) × 2.00 mm (inner diameter), 4 µm (Phenomenex Inc.) was used at 30°C. As a mobile phase, methanol/ a 10 mmol/l ammonium acetate solution/ acetic acid (700/300/2, v/v/v) was used, and a flow rate was set at 0.2 ml/min. An injection volume was set at 2 µl, a measurement time was set at 10 min, and the temperature of an autosampler was set at 4°C. As a mass spectrometer, Tandem Mass Spectrometer API 4000 (Applied Biosystems/MDS SCIEX Inc.) was used. As API interface, Turbo V (ESI probe) was used, and as Ionization mode, Positive ion detection mode was used. The precursor product ions in the Detection MRM were as follows: caffeine: m/z 195.1 → m/z 138.2 (170 msec/1 scan), tolbutamide: m/z 271.1 → m/z 74.3 (170 msec/ 1 scan), omeprazole: m/z 346.1 → m/z 198.2 (150 msec/ 1 scan), dextromethorphan: m/z 272.2 → m/z 215.3 (150 msec/ 1 scan), erythromycin: m/z 734.4 → m/z 158.4 (150 msec/ 1 scan), and phenytoin m/z 253.1 → m/z 182.3 (150 msec/ 1 scan).

### 1-5-3. Preparation of samples for addition calibration curve

5 µl aliquots of the serum of the control mouse were dispensed in Eppendorf tubes, and 100 µl of standard solutions of substances to be measured (mixtures of 5 compounds) were added thereto, and solutions with final concentrations of 0.1, 0.5, 1, 5, 10, 50, 100, 500 and 1000 ng/ml were prepared. Then, 10 µl of 1 µg/ml of I.S. standard solution was added thereto, and the mixtures were thoroughly mixed by an ultrasonic treatment and a vortex mixer. The resulting mixtures were centrifuged (15,000 rpm, 5 min, room temperature), and the resulting supernatants were subjected to centrifugal filtration (10,000 rpm, 2 min, room temperature) with a filter, and the resulting filtrates were used as injection samples for LC-MS/MS.

### 1-5-4. Preparation of samples to be measured

To Eppendorf tubes in which 5 µl of samples to be measured (serum) were contained, 100 µl of methanol was added, and 10 µl of 1 µg/ml of I.S. standard solution was added thereto, and then, the mixtures were thoroughly mixed by an ultrasonic treatment and a vortex mixer. The resulting mixtures were centrifuged (15,000 rpm, 5 min, room temperature), and the resulting supernatants were subjected to centrifugal filtration (10,000 rpm, 2 min, room temperature) with a filter, and the resulting filtrates were used as injection samples for LC-MS/MS.

### 2. Test results

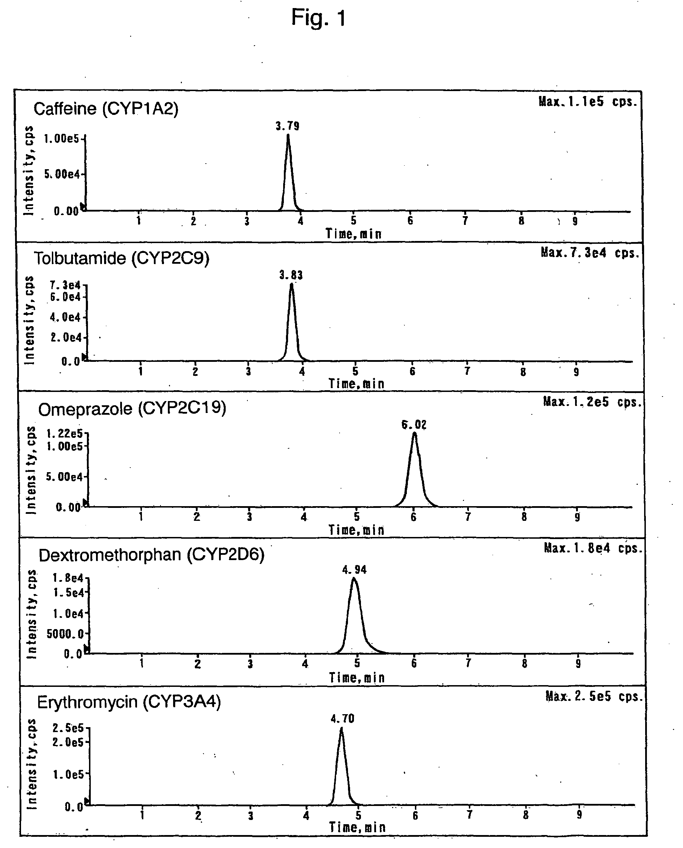

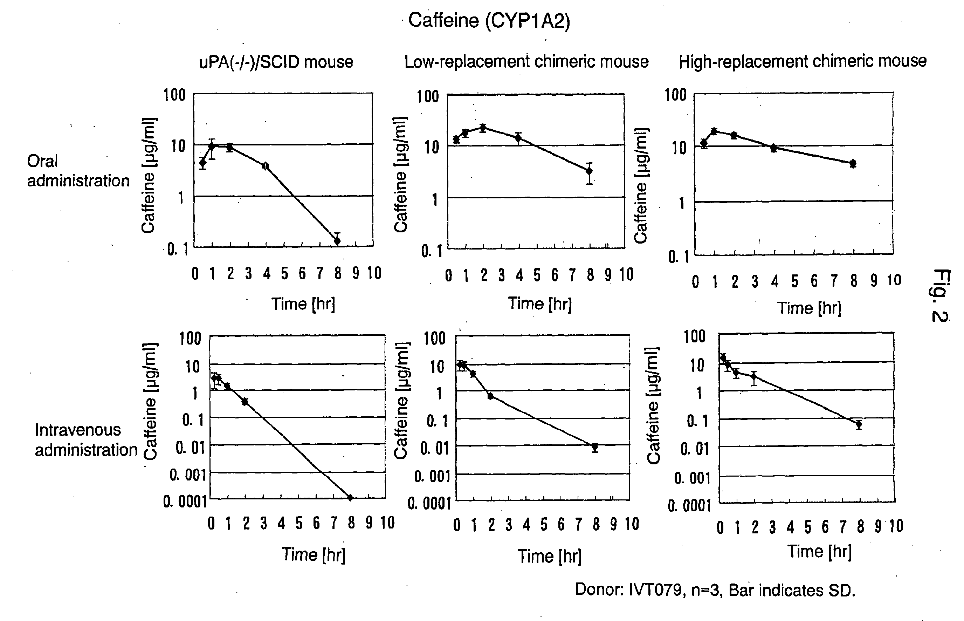

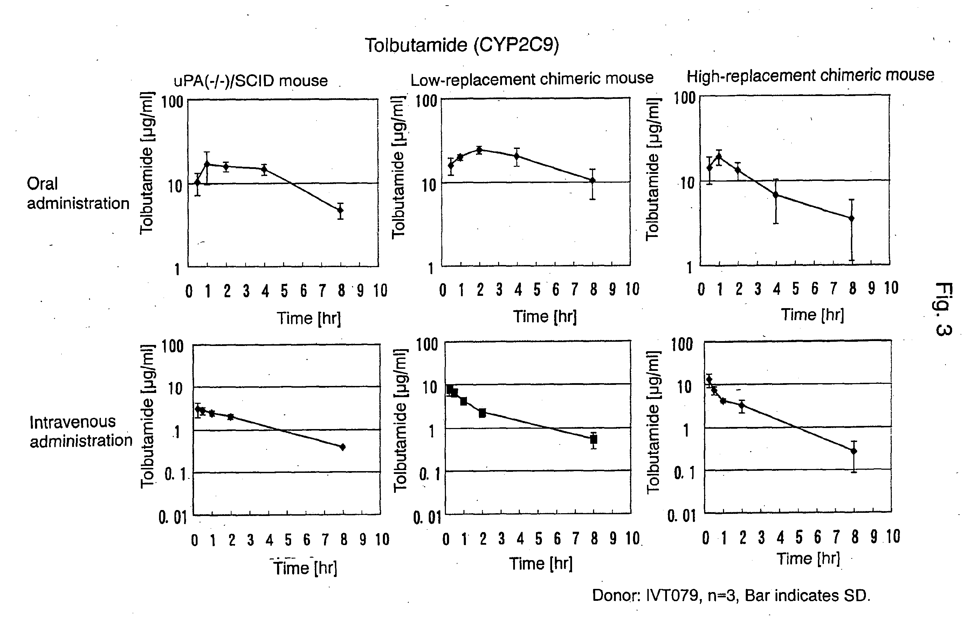

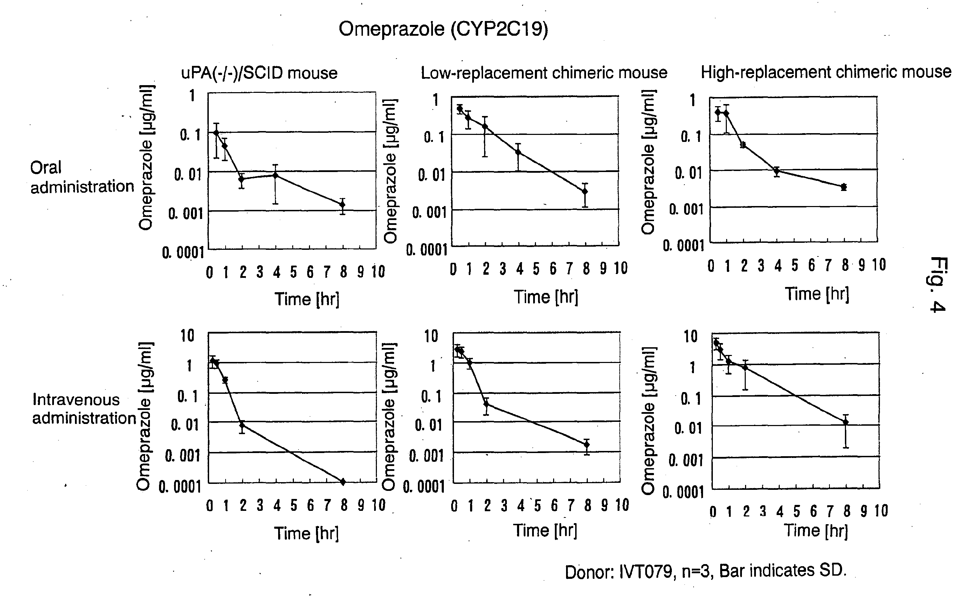

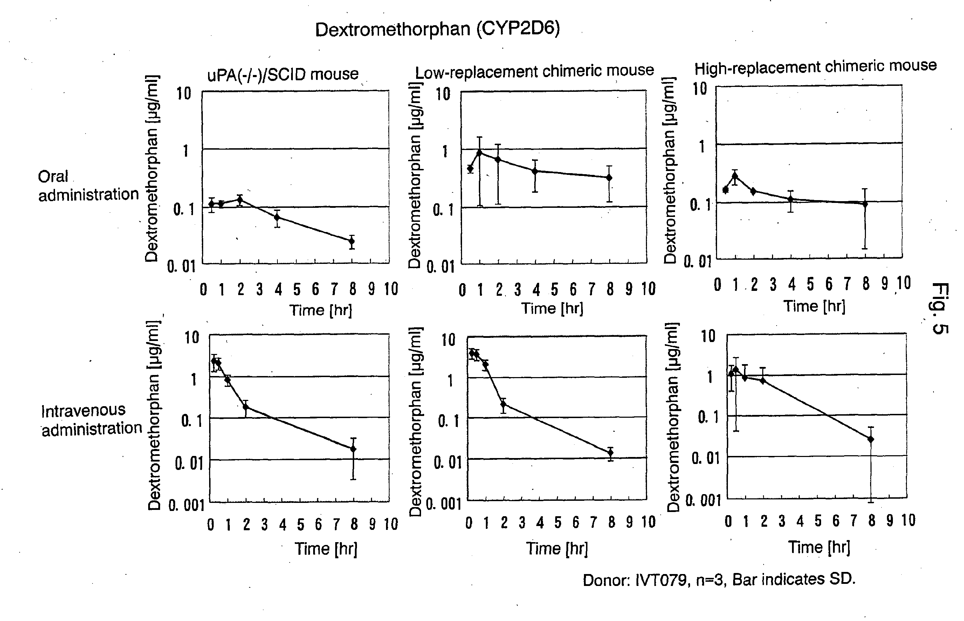

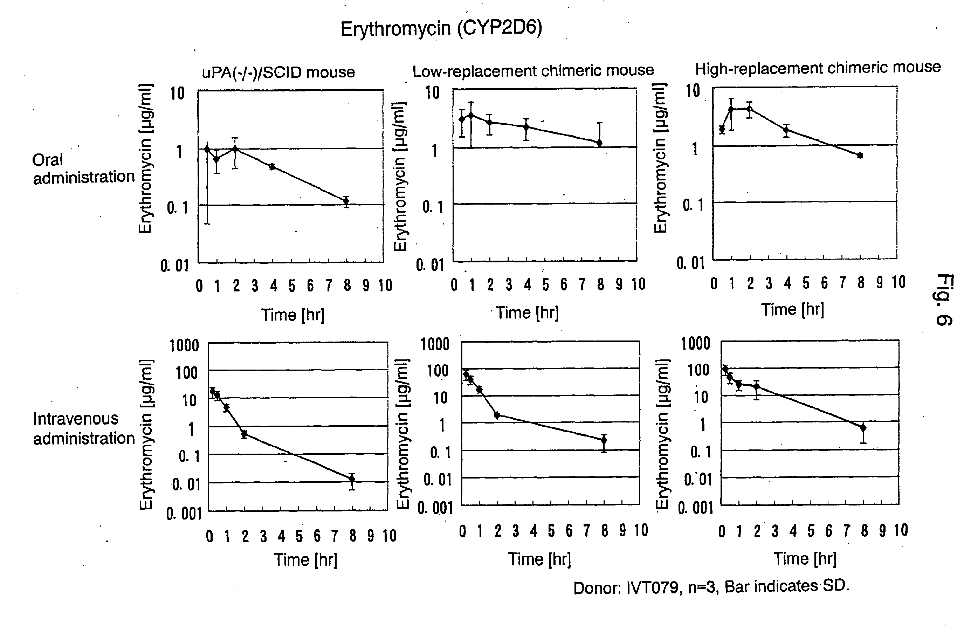

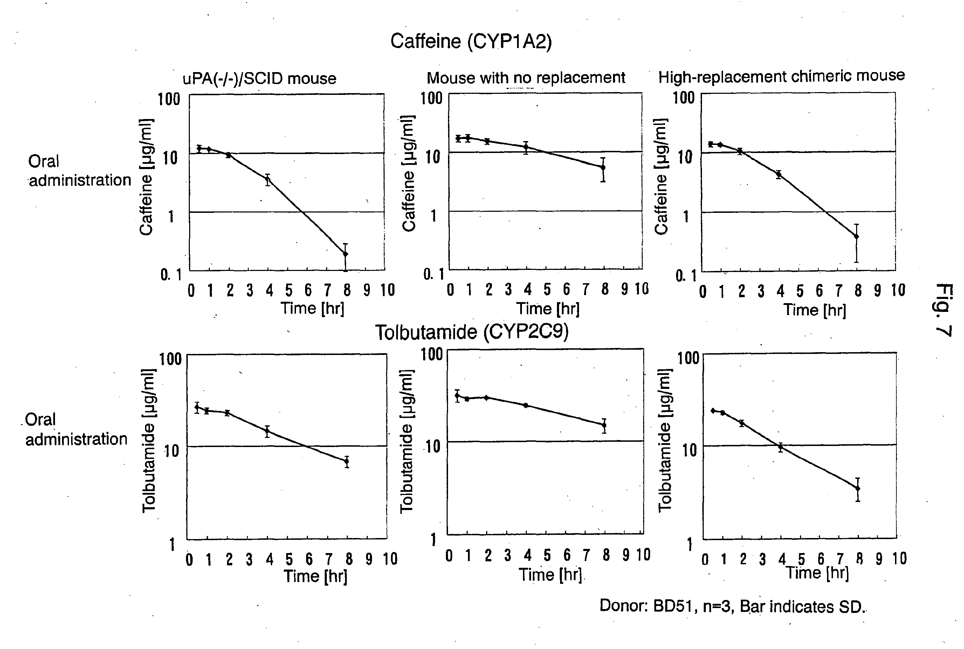

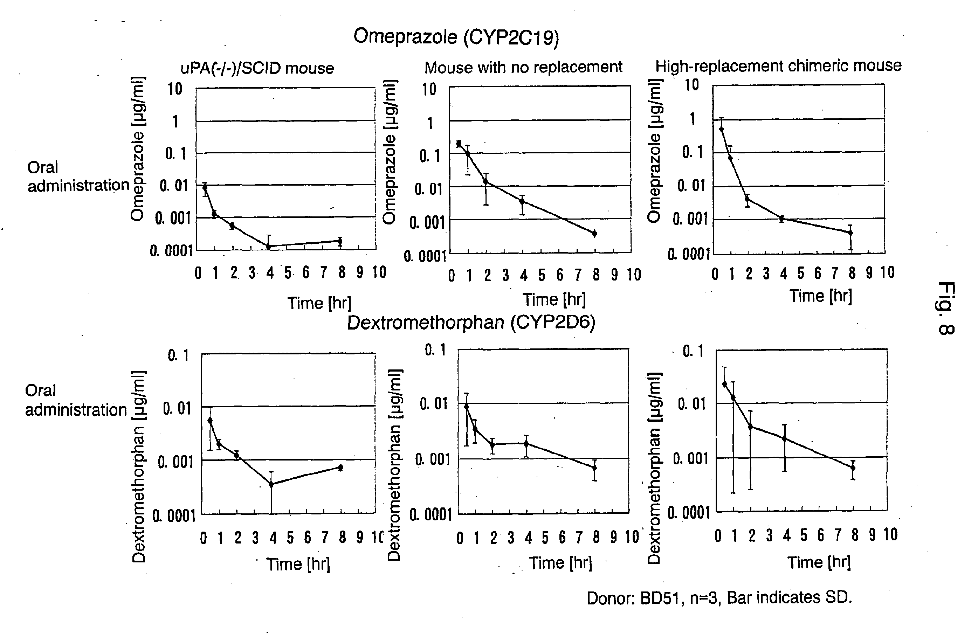

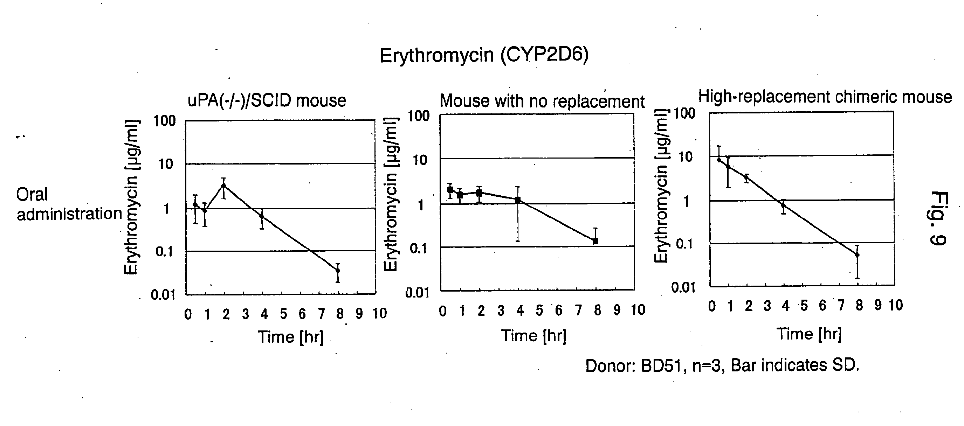

The determined values of the drug concentration in the serum were calculated from the peak area ratio obtained by the LC-MS/MS measurement of the samples for addition calibration curve. Fig. 1 shows examples of peaks obtained by LC-MS/MS measurement. From the determined values, the relationships between the blood collection time and the concentration of the administered drug in the serum are shown in graphs (Figs. 2 to 9). From the graphs, the peak serum concentration (Cₘₐₓ), half-life (t1/2), area under the curve (AUC), bioavailability and clearance were analyzed, and pharmacokinetics in human was predicted. The results are shown in Tables 1, 2, and 3.
Almost all the AUC associated with CYP was lower in the high-replacement chimeric mice than in the low-replacement chimeric mice. In the chimeric mice using IVT079 as the donor hepatocytes, the AUC in the case where tolbutamide (CYP2C9) and dextromethorphan (CYP2D6) were administered was significantly lower in the high-replacement chimeric mice than in the low-replacement chimeric mice. Further, also in terms of the bioavailability, similar results were obtained. In the chimeric mice in which BD51 was used as the donor hepatocytes, the AUC in the case where caffeine (CYP1A2) and tolbutamide (CYP2C9) were administered was significantly lower in the high-replacement chimeric mice than in the low-replacement chimeric mice.

**Table 1**

| | | Oral administration | | | | | Intravenous administration | | | |
|---|---|---|---|---|---|---|---|---|---|---|
| | | Dose (mg/kg) | Cₘₐₓ (µg/ml) ) | t1/2 (hr) | | AUC (µg/ml·hr) | Dose (mg/kg) | t1/2 (hr) | | AUC (µg/ml.hr) |
| | | | | Distribution phase | Elimination phase | | | Distribution phase | Elimination phase | |
| Caffeine (CYP1A2) | uPA(-/-)/SCID mouse | 5.00 | 8.8 | 2.45^{#1} | 0.82^{#2} | 33.2 | 0.500 | 0.609^{#3} | 0.505^{#10} | 4.07 |
| | Low-replacement chimeric mouse | | 21.4 | 2.16^{#3} | - | 97.5 | | 0.809^{#3} | 1.036^{#10} | 2.78 |
| | High-replacement chimeric mouse | | 18.9 | 3.45^{#4} 3.45^{#4} | - | 79.4 | | 0.437^{#3} 0.437^{#3} | 0.961^{#10} 0.961^{#10} | 7.11 |
| Tolbutamide (CYP2C9) | uPA(-/-)/SCID mouse | 1.67 | 16.9 | 15.16^{#1} | 2.42^{#2} | 95.3 | 0.167 | 2.84^{#3} | 2.56^{#10} | 11.5 |
| | Low-replacement chimeric mouse | | 24.8 | 4.7^{#3} | - | 144.0 | | 0.982^{#3} | 2.94^{#10} | 16.8 |
| | High-replacement chimeric mouse | | 90.0 | 2.25^{#1} | 4.3^{#2} | 68.6 | | 0.865^{#3} | 1.7^{#10} | 20.6 |
| Omeprazole (CYP2C19) | uPA(-/-)/SCID mouse | 1.67 | 0.094 | 0.38^{#3} | 2.74^{#3} | 0.114 | 0.167 | 0.24^{#3} | 0.971^{#10} | 0.84 |
| | Low-replacement chimeric mouse | | 0.485 | 1.02^{#8} | - | 0.792 | | 0.285^{#3} | 1.3^{#10} | 2.50 |
| | High-replacement chimeric mouse | | 0.397 | 4.92^{#8} | 1.03^{#4} | 0.582 | | 1.55^{#3} | 1^{#10} | 5.93 |
| Dextromethorphan (CYP2D6) | uPA(-/-)/SCID mouse | 0 500 | 0.130 | 2.45^{#3} | - | 0.58 | 0.0500 | 0.47^{#3} | 1.79^{#10} | 2.61 |
| | Low-replacement chimeric mouse chimeric mouse | | 0.836 | 2.88^{#1} | 9.98^{#2} | 3.65 | | 0.414^{#3} | 1.5^{#10} | 4.60 |
| | High-replacement chimeric mouse | | 0.276 | 1.15^{#8} | 10.36^{#3} | 1.02 | | 3.15^{#3} | 1.23^{#10} | 4.03 |
| Erythromycin (CYP3A4) | uPA(-/-)ISCID mouse | 25.0 | 1.0 | 1.93^{#3} | - | 4.08 | 2.50 | 0.35^{#3} | 1.1^{#10} | 13.8 |
| | Low-replacement chimeric mouse | | 3.5 | 4.38^{#4} 4.38^{#4} | - | 16.70 | | 0.342^{#3} 0.342^{#3} | 2.01^{#10} 2.01^{#10} | 47.8 |
| | High-replacement chimeric mouse | | 4.3 | 2.22^{#3} 2.22^{#3} | - | 17.20 | | 0.807^{#3} 0.807^{#3} | 1.17^{#10} 1.11^{#10} | 127 |

| | | | | | | | | | | |
|---|---|---|---|---|---|---|---|---|---|---|
| #1: 1-4 hr, #2: 4-8 hr, #3: 1-8 hr, #4: 1-8 hr, #5: 0.5-2 hr, #6: 0.5-8 hr, #7: 0.5-1 hr, #8: 1-2 hr, #9: 0.25-2 hr, #10: 2-8 hr Donor: IVT079 | | | | | | | | | | |

**Table 2**

| | Bioavailability | | | | |
|---|---|---|---|---|---|
| | CYP1A2 | CYP2C9 | CYP2C19 | CYP2D6 | CYP3A4 |
| uPA(-/-)/SCID mouse | 81.6 | 82.9 | 1.36 | 2.22 | 2.95 |
| Low-replacement chimeric mouse | 98.9 | 85.7 | 3.17 | 7.93 | 3.49 |
| High-replacement chimeric mouse | 55.4 | 33.3 | 0.98 | 2.53 | 1.35 |

| | | | | | |
|---|---|---|---|---|---|
| Donor: IVT079 | | | | | |

**Table 3**

| | | Dose (mg/kg) | Cₘₐₓ (µg/ml) | t1/2 (hr) | | AUC (µg/ml·hr) |
|---|---|---|---|---|---|---|
| | | | | Distribution phase | Elimination phase | |
| Caffeine (CYP1A2) | uPA(-/-)/SCID mouse | 5.00 | 11.8 | 1.26^{#1} | - | 57.6 |
| | Mouse with no replacement | | 17.0 | 4.58^{#1} 4.58^{#1} | - | 110 |
| | High-replacement chimeric mouse | | 13.6 | 1.43^{#1} | - | 65.7 |
| Tolbutamide (CYP2C9) | uPA(-/-)/SCID mouse | 1.67 | 25.7 | 7.02^{#2} | 3.45^{#3} | 150 |
| | Mouse with no replacement | | 30.7 | 18.2^{#2} 18.2^{#2} | 5.87^{#3} 5.87^{#3} | 208 |
| | High-replacement chimeric mouse | | 22.0 | 2.67^{#1} | - | 115 |
| Omeprazole (CYP2C19) | uPA(-/-)/SCID mouse | 1.67 | 0.00795 | 0.18^{#4} | 0.91^{#5} | 0.00713 |
| | Mouse with no replacement | | 0.0940 | 0.83^{#1} | - | 0.223 |
| | High-replacement chimeric mouse | | 0,5080 | 0.21^{#2} | 1.80^{#3} | 0.322 |
| Dextromethorphan (CYP2D6) | uPA(-/-)/SCID mouse | 0.500 | 0.00552 | 0.88^{#6} | - | 0.0095 |
| | Mouse with no replacement | | 0.00845 | 0.68^{#2} | 3.98^{#3} | 0.0189 |
| | High-replacement chimeric mouse | | 0.0228 | 0.57^{#2} | 2.31 ^{#3} | 0.0404 |
| Erythromycin (CYP3A4) | uPA(-/-)/SCID mouse | 25.0 | 1.22 | 0.92^{#3} | - | 14.1 |
| | Mouse with no replacement | | 1.99 | 4.70^{#6} | 1.25^{#7} | 11.6 |
| | High-replacement chimeric mouse | | 8.05 | 1.02^{#1} | - | 213 |

| | | | | | | |
|---|---|---|---|---|---|---|
| #1: 0.5-8 hr, #2: 0.5-2 hr, #3: 2-8 hr, #4: 0.5-1 hr, #5: 1-4 hr, #6: 0.5-4 hr, #7: 4-8 hr, Donor: BD51, Inhibitor: non | | | | | | |

From the above results, when the pharmacokinetics of various marker drugs were compared between the high-replacement chimeric mice and the low-replacement chimeric mice, in the case where the marker drugs of 2C19 and 3A4 were administered, a significant difference was not observed. However, it was found that depending on the donor hepatocytes used for transplantation, when the marker drugs of CYP1A2, CYP2C9 and CYP2D6 were administered, the metabolic rates thereof were lower in the low-replacement chimeric mice compared with the high-replacement chimeric mice. That is, it was revealed that in terms of CYP1A2, CYP2C9 or CYP2D6, the low-replacement chimeric mouse corresponds to PM (poor metabolizer) in human and the high-replacement chimeric mouse corresponds to EM (extensive metabolizer) in which the metabolic rate thereof is high. From this, in the development of a drug, a difference in the pharmacokinetics in the genetic polymorphism of a candidate drug which is metabolized mainly by CYP2C9 or CYP2D6 can be determined. Further, by selecting donor hepatocytes to be used for transplantation, it was contemplated that a difference in the pharmacokinetics in the genetic polymorphism of a candidate drug which is metabolized mainly by CYP2C19 can also be determined.
By examining the pharmacokinetics of a candidate drug metabolized mainly by CYP2C9, CYP2C19 or CYP2D6 in the high-replacement chimeric mice and the low-replacement chimeric mice, it becomes possible to make a go or no-go decision about the development of the candidate drug.

### Example 2

### Pharmacokinetic test using chimeric mouse under inhibition of CYP2D6

### 1. Materials and Methods

### 1-1. Test substance

In order to evaluate pharmacokinetics in human hepatocyte chimeric mice using marker drugs under the conditions in which CYP2D6 enzyme was inhibited by an (inhibitor of CYP2D6 enzyme), 5 compounds, i.e., caffeine (CYP1A2), tolbutamide (CYP2C9), omeprazole (CYP2C19), dextromethorphan (CYP2D6) and erythromycin (CYP3A4) which are marker drugs for cytochrome P450 (CYP) were used.

### 1-2. Animal used

As for the chimeric mice, chimeric mice produced in PhoenixBio Co., Ltd. were used. To be more specific, 3 mice in which human hepatocytes were engrafted and proliferated in the mouse liver were produced by transplanting human hepatocytes (BD51, purchased from BD Gentist Inc.) into the spleen of uPA(+/+)/SCID mice, which are mice having hepatic impairment and immunodeficiency.
Each of the mice was fed with water and feed and raised in the same manner as in Example 1. Incidentally, to the high-replacement chimeric mice, an injection solution of nafamostat mesilate (0.3 mg/0.2 ml/body) was intraperitoneally administered twice daily in the same manner as in Example 1.

### 1-3. Preparation of drug for inhibition

Paroxetine was dissolved in Milli-Q water (using a bath-type ultrasonic device) and prepared to give a final concentration of 6 mg/ml. The prepared drug was intraperitoneally administered to the mice at a dose of 5 µl/g b.w. (30 mg/kg b.w./day).

### 1-4. Preparation of marker drugs

The preparation of marker drugs was carried out in the same manner as in Example 1.

### 1-5. Administration method and serum collection method

To the high-replacement chimeric mice, paroxetine was intraperitoneally administered for 3 days. Then, in the same manner as in Example 1, 5 compounds were simultaneously administered orally by gavage, the blood was collected over time, and the serum was stored.

### 1-6. Determination and analysis of drugs in serum

The determination and analysis of drugs in the serum were carried out in the same manner as in Example 1.

### 1-6-1. Preparation of standard solutions of substances to be measured and I.S. standard solutions

The preparation of standard solutions of substances to be measured and I.S. standard solutions was carried out in the same manner as in Example 1.

### 1-6-2. Measurement device and measurement conditions (LC-MS/MS)

The measurement was carried out in the same manner as in Example 1.

### 1-6-3. Preparation of samples for addition calibration curve

The preparation of samples for addition calibration curve was carried out in the same manner as in Example 1.

### 1-6-4. Preparation of samples to be measured

The preparation of samples to be measured was carried out in the same manner as in Example 1.

### 2. Test results

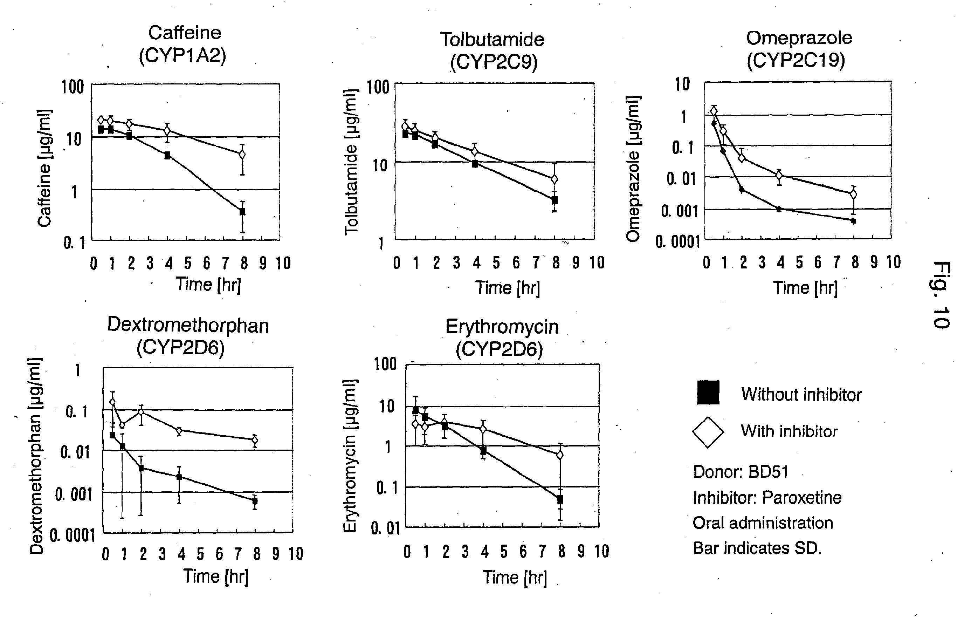

The determined values of the drug concentration in the serum were calculated from the peak area ratio obtained by the LC-MS/MS measurement of the samples for addition calibration curve. From the determined values, the relationships between the blood collection time and the concentration of the administered drug in the serum are shown in graphs (Fig. 10). From the graphs, Cₘₐₓ, t1/2 and AUC were analyzed.
The results are shown in Table 4. By the administration of paroxetine, the inhibition of the metabolic activities of CYP1A2, 2C19, 2D6 and 3A4 was observed. The metabolic activity of CYP2D6, which is said to be specifically inhibited by paroxetine, was particularly inhibited.

**Table 4**

| | | Dose(mg/kg) | Cₘₐₓ(µg/ml) | t1/2 (hr) | | AUC (µg/ml·hr) |
|---|---|---|---|---|---|---|
| | | | | Distribution phase | Elimination phase | |
| Caffeine (CYP1A2) | High-replacement chimeric mouse | 5.00 | 20.6 | 3.40^{#1} | - | 97.1 |
| Tolbutamide (CYP2C9) | | 1.67 | 28.2 | 3.31^{#1} | - | 115 |
| Omeprazole (CYP2C19) | | 1.67 | 1.31 | 0.30^{#2} | 1.55^{#3} | 0.964 |
| Dextromethorphan (CYP2D6) | | 0.500 | 0.156 | 1.44^{#4} | 5.29^{#5} | 0.358 |
| Erythromycin CYP3A4) | | 25.0 | 3.48 | 2.16^{#3} | - | 18.4 |

| | | | | | | |
|---|---|---|---|---|---|---|
| #1: 0.5-8 hr, #2: 0.5-2 hr, #3: 2-8 hr, #4: 0.5-4 hr, #5: 4-8 hr, Donor: BD51, Inhibitor: Paroxetine | | | | | | |

From the above results, by administering a test substance to the high-replacement chimeric mouse and examining the change in AUC of each marker substance, it is possible to examine the activity of induction or inhibition of the test substance. Therefore, by using the chimeric mouse, it is possible to predict drug interaction in human.

## Claims

1. A method of determining whether or not a candidate drug metabolized mainly by a drug-metabolizing enzyme, CYP2D6, CYP2C9 or CYP2C19 is metabolized in a subject deficient in CYP2D6, CYP2C9 or CYP2C19, which comprises the steps of:
(1) administering the candidate drug to a high-replacement chimeric mouse in which transplanted human hepatocytes account for 70% or more of the mouse liver, and a low-replacement chimeric mouse in which few human hepatocytes are engrafted, respectively; and
(2) measuring the time-course concentration of the candidate drug in serum collected from the high-replacement chimeric mouse and the low-replacement chimeric mouse,
and determining that the candidate drug which is metabolized significantly more quickly in the high-replacement chimeric mouse than in the low-replacement chimeric mouse is a substance which is not metabolized in the subject deficient in CYP2D6, CYP2C9 or CYP2C19.

2. A method of determining whether or not a candidate drug inducesor inhibits the activity of a drug-metabolizing enzyme, which comprises the steps of:
(1) administering the candidate drug to a high-replacement chimeric mouse;
(2) administering a plurality of marker compounds which are metabolized by a respective plurality of drug-metabolizing enzymes to the high-replacement chimeric mouse; and
(3) measuring the time-course concentrations of the marker compounds in serum collected from the high-replacement chimeric mouse,
and determining whether or not the candidate drug induces or inhibits the activity of any of the drug-metabolizing enzymes by the comparison with the concentrations of marker compounds in serum when the candidate drug is not administered.
